Europäisches Patentamt

European Patent Office

Office européen des brevets

(19)

(11) Veröffentlichungsnummer: **0 250 991 B1**

(12) **EUROPÄISCHE PATENTSCHRIFT**

(45) Veröffentlichungstag der Patentschrift: **27.01.93**

(21) Anmeldenummer: **87108517.1**

(22) Anmeldetag: **12.06.87**

(51) Int. Cl.⁵: **G01N  33/66**, C12Q 1/26, //C12Q1/54,G01N33/52

(54) **Verfahren zur spezifischen Bestimmung des Serumfructosamingehalts sowie hierfür geeignetes Reagenzgemisch.**

(30) Priorität: **21.06.86 DE 3620817**

(43) Veröffentlichungstag der Anmeldung:
**07.01.88 Patentblatt  88/01**

(45) Bekanntmachung des Hinweises auf die
Patenterteilung:
**27.01.93 Patentblatt  93/04**

(84) Benannte Vertragsstaaten:
**AT BE CH DE ES FR GB GR IT LI LU NL SE**

(56) Entgegenhaltungen:
EP-A- 0 004 857          EP-A- 0 014 898
EP-A- 0 016 962          EP-A- 0 148 950
EP-A- 0 158 964          EP-A- 0 215 170
DE-A- 2 910 737          GB-A- 2 084 726

CLINICA CHIMICO ACTA, Vol. 127, 1982, Elsevier Biomedical Press, Amsterdam, NL; R.N. JOHNSON et al.: "Fructosamine: a new cosylprotein. An index of diabetic control", Seiten 87-95;

CHEMICAL ABSTRACTS, Band 88, Nr. 17, 24. April 1978, Seite 226, Spalte 1, Zusammenfassung Nr. 117280z; M. HIGUCHI et al.: "An improved Lowry procedure by using chloramine-T under neutral or alkaline conditions";

(73) Patentinhaber: **BOEHRINGER MANNHEIM GMBH**
**Sandhofer Strasse 116**
**W-6800 Mannheim 31(DE)**

(72) Erfinder: **Siedel, Joachim, Dr.rer.nat.**
**Bahnhofstr. 6**
**W-8131 Bernried(DE)**
Erfinder: **Ziegenhorn, Joachim, Dr.rer.nat.**
**Ina Seidel-Weg 1**
**W-8130 Starnberg(DE)**
Erfinder: **Schellong, Lieselotte**
**Schluderstr. 14**
**W-8000 München 19(DE)**
Erfinder: **Vogt, Bernd, Dr.med.**
**Mozartstr. 1**
**W-8132 Tutzing(DE)**

**Beschreibung**

Die vorliegende Erfindung betrifft ein Verfahren zur spezifischen Bestimmung des Serumfructosamingehalts in Blut oder von Blut abgeleiteten Proben unter Vermeidung des Proteinmatrixeffektes.

Unter Serumfructosamingehalt versteht man den Gesamtgehalt an nichtenzymatisch glycosilierten Serumproteinen. Diese entstehen dadurch, daß Serumglucose über ihre Carbonylgruppe mit freien Proteinaminoresten Schiff'sche Basen bildet. Diese gehen anschließend durch Amadori-Umlagerung in Fructosamine mit stabiler Ketoamin-Bindung über. Der Reaktionsmechanismus wurde z. B. von E. Schleicher und O. H. Wieland in J. Clin. Chem. Clin. Biochem. (1981) 19, 81 - 87 näher untersucht.

Die Halbwertzeit des Serumfructosamins ist wegen der Stabilität der Ketoamin-Bindung praktisch identisch mit der der Serumproteine, deren Halbwertzeit im Durchschnitt ca. 21 Tage beträgt. Entsprechende Untersuchungen wurden von L. Y. Seng und M. J. Staley in J. Clin. Chem. (1986) 32, 560 veröffentlicht.

Das Ausmaß der Fructosamin-Bildung ist proportional dem Blutglucose-Spiegel. Dieser kann bekanntermaßen bei Diabetikern, besonders bei ungenügender diätetischer und medikamentöser Stoffwechseleinstellung starken Schwankungen, verbunden mit ausgeprägten pathologischen Erscheinungen, unterliegen.

Die Blutglucose-Bestimmung gibt dem Arzt nur Auskunft über die zum Zeitpunkt der Blutentnahme vorhandene Stoffwechsellage. Eine Langfristkontrolle über die Stoffwechsellage der letzten 120 Tage ist dagegen mit der Bestimmung des glycosilierten Hämoglobins (HbA$_1$) möglich. Die Messung des Serumfructosamins eignet sich nun gerade wegen dessen Halbwertzeit dazu, die Stoffwechselführung von Diabetikern durch Lebenshaltung und therapeutische Maßnahmen rückwirkend über einen mittelfristigen Zeitraum von ca. 3 Wochen zu ermitteln. In Verbindung mit den etablierten klinisch diagnostischen Parametern Blutglucose sowie glycosiliertes Hämoglobin (HbA$_1$) ließe sich deshalb mit einer zuverlässigen, spezifischen und praktikablen Methode zur Serumfructosamin-Bestimmung das Diagnosearsenal zur Überwachung von Diabetikern um einen wertvollen Mittelfristparameter erweitern.

Ein vom Prinzip her einfach durchführbares Verfahren zur Serumfructosamin-Bestimmung wurde von Baker in EP-C 0 085 263 und von Johnson et al. in Clin. Chim. Acta (1982) 127, 87 - 95 beschrieben. Es beruht darauf, daß die Ketoaminform in wässrigem, alkalischem Medium in eine Enolform übergeht, die reduzierend auf Tetrazoliumsalze, wie z. B. Nitrotetrazoliumblau wirkt und dabei einen Formazanfarbstoff liefert. Das Ausmaß der in einem definierten Zeitintervall bei 37° C photometrisch gemessenen Farbstoffbildung ist der Menge an vorliegendem Fructosamin proportional.

Dieser Test ist bei Verwendung von Serum als Probenmaterial störanfällig, denn natürliche Serumbestandteile, wie Bilirubin und Harnsäure, wirken ebenfalls auf Tetrazoliumsalze reduktiv. Auch Medikamente, wie z. B. α-Methyldopa, Medikamentabbauprodukte, wie z. B. Gentisinsäure, die ein Metabolit der Acetylsalicylsäure ist, sowie Ascorbinsäure führen je nach Konzentration im Serum zu verfälschten Meßergebnissen.

EP-A-0215 170 beschreibt einen Endpunkttest für Fructosamin, bei dem zur Beseitigung störender unspezifisch reduzierender Probenbestandteile eine Reihe möglicher Maßnahmen vorgeschlagen werden. Neben der bevorzugten Maßnahme der Probeninkubation mit einer NaOH-Lösung bei pH 10 während mindestens 30 Minuten werden auch eine Reihe weniger bevorzugter Maßnahmen erwähnt wie z. B. Probeninkubation bei höherer Temperatur, Entsalzen der Probelösung über Dialyse oder Gelpermeationschromatographie oder Zugabe von enzymatischen oder nicht enzymatischen Oxidationsmitteln. Serumfructosamin-Bestimmungen leiden bisher außerdem unter Störungen, die auf dem von Probe zu Probe variierenden Gesamtproteingehalt beruhen. Sie führen zu Meßwertschwankungen und verringern dadurch die Empfindlichkeit des Bestimmungsverfahrens. Diese Störungen sind als Matrixeffekte bekannt. Besonders bemerkbar macht sich dieser Effekt bei Zugabe zusätzlichen Proteins, wie dies z. B. bei der Herstellung von Standardlösungen üblicherweise der Fall ist. Steigende Proteinmengen verlangsamen die Reaktion zwischen Fructosamin und Farbreagenz (vergleiche E. J. Hindle et al., Ann. Clin. Biochem. 22 - (1985), S. 84 - 89).

Weitere Schwierigkeiten treten bei der Fructosamin-Bestimmung in hyperlipämischen Seren auf. Im allgemeinen ist, um ein auch bei niedrigen Fructosaminkonzentrationen in der Probe noch ausreichend großes Meßsignal zu erhalten, ein Probe-/Reagenzvolumenverhältnis von 0,1 erforderlich. Bei überhöhten Triglyceridkonzentrationen macht sich jedoch bei einem so hohen Probenanteil die resultierende Trübung des Testansatzes negativ bei der photometrischen Messung bemerkbar. Die Fructosamin-Bestimmung wird erheblich erschwert oder sogar verhindert.

GB-A-2084 726 beschreibt allgemein eine Möglichkeit zur Beseitigung von Trübungen in biologischen Proben mit einem nichtionischen Detergenz und Cholesterinesterase oder Lipase. Ein Hinweis auf einen Fructosamintest oder auf die Beseitigung eines diesen störenden Proteinmatrixeffektes ist in dieser Anmeldung nicht enthalten.

Es besteht weiterhin Bedarf an einem Verfahren zur spezifischen Bestimmung des Serumfructosamingehaltes in Blut oder von Blut abgeleiteten Proben, das die oben genannten Nachteile nicht aufweist. Aufgabe der vorliegenden Erfindung war es, ein solches Verfahren bereitzustellen.

Gelöst wird diese Aufgabe durch die in den Patentansprüchen gekennzeichnete Erfindung. Das erfindungsgemäße Verfahren zur spezifischen Bestimmung des Serumfructosamingehaltes in Blut oder von Blut abgeleiteten Proben durch Umsetzung mit einem Redoxfarbreagenz und Messung der dadurch bewirkten Farbänderung unter Vermeidung des Proteinmatrixeffektes, ist dadurch gekennzeichnet, daß vor der Farbreaktion die Probe mit einem oder mehreren enzymatischen oder/und nichtenzymatischen Oxidationsmitteln, Lipase und einem oder mehreren Detergenzien bei einem pH-Wert zwischen 6 und 9 behandelt, anschließend ein pH-Wert zwischen 10 und 12 eingestellt und das Farbreagenz zugegeben wird.

Vorteilhaft werden als enzymatische Oxidationsmittel Ascorbat-Oxidase, Bilirubin-Oxidase oder/und Uricase und als nichtenzymatische Oxidationsmittel Hypochlorit oder eine hypochloritliefernde Verbindung eingesetzt. Als hypochloritliefernde Verbindung ist besonders N-Chlor-p-toluol-sulfamid (Chloramin T) geeignet. Die Verbindung spaltet bei Berührung mit Wasser langsam unterchlorige Säure ab. Letztere ist im Sauren bis Neutralen, nicht dagegen im Alkalischen, ein wirksames Oxidationsmittel. Besonders vorteilhaft hat es sich erwiesen, wenn zusätzlich Peroxidase oder/und Katalase zugegeben werden.

Überraschenderweise wurde festgestellt, daß durch Zusatz eines oder mehrerer enzymatischer oder/und nichtenzymatischer Oxidationsmittel zu der zu bestimmenden Probe, Störungen durch sämtliche störende reduzierend wirkende Bestandteile beseitigt werden, d. h. die oben genannten Oxidationsmittel oxidieren nicht nur Bilirubin, Harnsäure oder Ascorbinsäure, sondern sie beseitigen auch Störungen durch andere reduzierend wirkende Stoffe, wie z. B. Medikamente sowie deren Abbauprodukte. In Gegenwart des oder der Oxidationsmittel werden solche Substanzen im therapeutischen Konzentrationsbereich eliminiert oder ihre Konzentration auf ein analytisch nicht mehr ins Gewicht fallendes Ausmaß herabgesetzt. Eventuell vorhandene Peroxidase kann mit gegebenenfalls entstehendem Wasserstoffperoxid zusätzliche oxidative Abbauprozesse bewirken, was die Wirksamkeit des oder der Oxidationsmittel noch zusätzlich erhöht. Gegebenenfalls zugesetzte Katalase dient vor allem zur Beseitigung überschüssigen Wasserstoffperoxids. Durch Zusatz eines geeigneten Detergens, vorteilhafterweise eines kationischen Detergens, beispielsweise Oxyethylalkylammoniumphosphat (z. B. Dehyquart$^R$SP der Fa. Henkel) kann überraschenderweise die oxidative Wirkung erhöht werden. Ganz besonders wirksam ist ein Gemisch von enzymatischen oder/und nichtenzymatischen Oxidationsmitteln, wenn es Chloramin T und gleichzeitig ein kationisches Detergens, beispielsweise Oxyethylalkylammoniumphosphat (z. B. Dehyquart$^R$SP), enthält. Die Konzentration an kationischem Detergens reicht von 0,5 bis 4 Vol.-%. Als bevorzugter Konzentrationsbereich hat sich für Dehyquart$^R$SP 1 bis 2 Vol-% erwiesen.

Insbesondere für die Untersuchung lipämischer Proben hat es sich als zweckmäßig erwiesen, dem oder den Oxidationsmitteln gegebenenfalls Lipase, gegebenenfalls zusammen mit einem oder mehreren Detergentien oder/und Salzen starker Säuren zuzusetzen. Mit Hilfe eines solchen Gemisches gelingt es, trübungsverursachende Stoffe soweit zu eliminieren, daß die anschließende Farbmessung nicht mehr beeinträchtigt ist.

Überraschenderweise werden mit Hilfe der vorgenannten Substanzen zur Beseitigung störender Bestandteile auch die als Matrixeffekte bekannten Meßwertschwankungen durch variierende Gesamtproteinmengen in der Probe weitgehend unterdrückt bzw. vollständig beseitigt.

Zur Beseitigung des Proteinmatrixeffektes hat es sich als zweckmäßig erwiesen, die Probe mit einer Lösung zu inkubieren, welche das oder die enzymatischen oder/und nichtenzymatischen Oxidationsmittel, Lipase sowie ein oder mehrere Detergentien sowie gegebenenfalls Salze starker Säuren in einem geeigneten nichtreduzierenden Puffer enthält. Zur Herstellung dieser Lösung sind sämtliche Puffersubstanzen geeignet, die selbst nicht reduzierend wirken und deren Pufferwirkung bei ungefähr neutralem pH-Wert liegt. Vorteilhaft hat sich ein pH-Bereich von pH 6 - 9, vorzugsweise pH 7 - 8,5, ganz besonders bevorzugt pH 7,5 - 8,0, erwiesen. Die Pufferkonzentration beträgt vorzugsweise 10 - 100 mmol/l, ganz besonders bevorzugt 20 - 70 mmol/l. Als besonders vorteilhaft hat sich wässriger Kaliumphosphatpuffer erwiesen.

Die Konzentration der zugesetzten Enzyme richtet sich nach der Konzentration der zu beseitigenden störenden Verbindungen. Üblicherweise liegen diese Enzymkonzentrationen zwischen 0,01 und 10 000 U/ml. Bevorzugte Konzentrationsbereiche sind beispielsweise für

| Uricase | 1 - 15 U/ml |
|---|---|
| Bilirubin-Oxidase | 0,05 - 5 U/ml |
| Ascorbat-Oxidase | 2 - 20 U/ml |
| Lipase | 0,5 - 5 U/ml |
| Peroxidase | 0,5 - 5 U/ml |
| Katalase | 100 - 10 000 U/ml |

Besonders bevorzugte Konzentrationsbereiche dieser Enzyme sind für

| Uricase | 2 - 10 U/ml |
|---|---|
| Bilirubin-Oxidase | 0,1 - 1 U/ml |
| Ascorbat-Oxidase | 5 - 15 U/ml |
| Lipase | 1 - 3 U/ml |
| Peroxidase | 1 - 3 U/ml |
| Katalase | 500 - 2000 U/ml. |

Auch die Konzentration des zugesetzten Hypochlorits bzw. der hypochloritliefernden Verbindungen richtet sich nach der Konzentration der zu beseitigenden störenden Verbindungen. Üblicherweise werden Hypochlorit und hypochloritliefernde Verbindungen in Konzentrationen von 50 bis 600 μmol/l, vorzugsweise 150 bis 300 μmol/l eingesetzt.

Detergentien können außer kationischen auch anionische oder nichtionische Detergentien sein. Als anionische Detergentien werden Alkali- oder Erdalkalisalze von Gallensäuren und ihrer Konjugate bevorzugt. Vorteilhafte Konzentrationen anionischer Detergentien liegen zwischen 2 und 10 mmol/l. Als besonders günstig hat sich Natriumcholat in Konzentrationen von 4 bis 6 mmol/l erwiesen.

Als nichtionische Detergentien steht eine breite Palette von Detergentien zur Auswahl. Als geeignet haben sich vor allem lineare oder verzweigtkettige Alkyl- oder Alkylaryl-Alkohol-Polyglycoläther mit 8 bis 20 C-Atomen im Alkoholteil und 4 bis 15 Glycoleinheiten pro Molekül erwiesen. Eine besonders vorteilhafte aktivierende Wirkung üben lineare und verzweigtkettige Alkyl-Alkohol-Polyglycoläther mit 8 bis 12 C-Atomen im Alkoholteil und 4 bis 8 Glycoleinheiten pro Molekül aus.

Für das erfindungsgemäße Verfahren zur spezifischen Bestimmung des Serumfructosamingehaltes sind nichtionische Detergentien geeignet, die bezüglich der Struktur des Alkoholteils einheitlich oder ein Gemisch aus mehreren, hinsichtlich der Struktur des Alkoholteils unterschiedlichen Polyglycoläthern sein können. Besonders bevorzugt wird ein Gemisch aus einem Isodecanolpolyglycoläther mit durchschnittlich vier Glycoleinheiten pro Molekül (Oxetal[R] ID 104 der Firma Zschimmer & Schwarz, Lahnstein) und einem n-Decanolpolyglycoläther mit durchschnittlich 6 Glycoleinheiten pro Molekül (Produkt RT 240[R] der Firma Zschimmer & Schwarz, Lahnstein). Die Konzentration an nichtionischem Detergenz, die erfindungsgemäß zur Beseitigung des Proteinmatrixeffektes eingesetzt wird, reicht von 0,05 bis 15 Gew. %. Als bevorzugter Konzentrationsbereich hat sich für Oxetal[R] ID 104 0,1 bis 1 %, als besonders vorteilhaft 0,2 bis 0,5 % erwiesen. Produkt RT 240[R] kann in Konzentrationen von 1 bis 10 %, besonders vorteilhaft 2 bis 5 %, eingesetzt werden.

Die trübungsbeseitigende Wirkung kann durch höhere Ionenstärken in der Reaktionslösung noch verstärkt werden. Hierfür haben sich Zusätze von Salzen starker Säuren, die auch im alkalischen pH-Bereich in Lösung bleiben, als günstig erwiesen. Bevorzugt werden Alkali- oder Erdalkalisalze von Salz- oder Schwefelsäure. Besonders bevorzugt werden Kalium- oder Natriumchlorid eingesetzt. Die Konzentration der zugesetzten Salze starker Säuren reichen von 20 bis 100 mmol/l. Besonders bevorzugt werden Salze in der Konzentration von 40 bis 60 mmol/l zugesetzt.

Die Beseitigung unspezifisch reduzierend wirkender und trübungsverursachender Probenbestandteile erfolgt bei Temperaturen zwischen 25 und 40° C, vorzugsweise bei 37° C über einen Zeitraum von 1 bis 15 Minuten, vorzugsweise 2 bis 6 Minuten. Die zu wählende Zeitspanne der Inkubation ist abhängig von der Menge an unspezifisch reduzierend wirkenden und trübungsverursachenden Probenbestandteilen und der zu ihrer Beseitigung eingesetzten Menge an enzymatischen oder/und nichtenzymatischen Oxidationsmitteln, Lipase und Detergenzien.

Da die Inkubation zur Beseitigung unspezifisch reduzierend wirkender und trübungsverursachender Probenbestandteile wegen des pH-Optimums der verwendeten Enzyme bei ungefähr neutralem pH-Wert durchgeführt wird, die Farbreaktion zwischen Farbreagenz und Fructosamin aber bei einem pH-Wert zwischen 10 und 12 verläuft, ist es erforderlich, nach erfolgter Inkubation umzupuffern. Die Erhöhung des pH-Wertes erfolgt mittels eines Puffers, dessen pH-Wert etwas über dem einzustellenden pH-Wert liegt. Ein Puffer mit einem pH-Wert zwischen pH 10,5 und 12,5, besonders bevorzugt zwischen pH 10,7 und 12,2 ist besonders zweckmäßig. Vorteilhafterweise wird hierfür ein Carbonatpuffer eingesetzt, dessen Konzentration 150 bis 300 mmol/l, besonders bevorzugt 180 bis 220 mmol/l beträgt.

In an sich bekannter Weise kann durch Zugabe eines Farbreagenzes im alkalischen Bereich die reduzierende Wirkung des Fructosamins sichtbar gemacht werden. Vorzugsweise wird hierfür ein Tetrazoliumsalz eingesetzt, dessen Formazanfarbstoffbildung visuell oder photometrisch verfolgt werden kann. Als Tetrazoliumsalze werden diejenigen bevorzugt, die in "Methods of Enzymatic Analysis" (H. U. Bergmeyer, Hrsg., 3. Ausgabe, Verlag Chemie Weinheim 1983, Band I, Seite 200) beschrieben sind. Besonders geeignetz sind Nitrotetrazoliumblau (NBT) oder 3-(4',5'-Dimethylthiazolyl-2-)-2,4-diphenyl-tetrazoliumbromid (MTT). Das Farbreagenz kann sowohl nach der Umpufferung als auch gleichzeitig mit dem Puffer dem Testansatz zugegeben werden. Hierfür hat es sich als vorteilhaft erwiesen, das Farbreagenz in dem für die Umpufferung benötigten Puffer zu lösen. Für Tetrazoliumsalze haben sich Konzentrationen von 0,2 bis 2 mmol/l als günstig, 0,4 bis 1,5 mmol/l als besonders günstig erwiesen.

Zur Umpufferung nach der Beseitigung unspezifisch reduzierend wirkender und trübungsverursachender Probenbestandteile wird soviel Puffer zugesetzt, daß der pH-Wert des Testansatzes bei Anwesenheit des Farbreagenzes einen pH-Wert zwischen 10 und 12, vorzugsweise 10,3 bis 10,6 aufweist.

Die umgepufferte Lösung wird bei Temperaturen zwischen 25 und 40° C, vorzugsweise bei 37° C inkubiert. Die Farbänderung beruhend auf der Reduktion des Farbreagenzes wird photometrisch in einem definierten Zeitintervall, vorzugsweise 1 bis 15 Minuten nach Umpufferung verfolgt. Eine erste Messung wird 1 bis 10 Minuten nach der Umpufferung und eine letzte Messung 2 bis 15 Minuten nach der Umpufferung durchgeführt. Je nach Erfordernis können zwei oder mehr Messungen durchgeführt werden. Die zeitlichen Abstände zwischen zwei Messungen sind variabel. Sie können je nach apparativer Ausstattung gewählt werden, wobei sie im Bereich weniger Sekunden als auch einiger Minuten liegen können.

In manchen Fällen ist es zweckmäßig, die erhaltenen Meßwerte mit denen einer Standardlösung zu vergleichen. Geeignete Standardlösungen sind bekannt. Beispielsweise kann hierzu der von Johnson et al. in Clin. Chim. Acta (1982) 127, 87 - 95 beschriebene Standard verwendet werden, der auf einer Matrix aus Humanalbumin mit definierten Zusätzen eines synthetischen Fructosamins basiert. Als synthetisches Fructosamin wird 1-Desoxy-1-morpholino-fructose (DMF) verwendet. Die ermittelte Serumfructosamin-Konzentration in der Probe wird bei Verwendung dieses Standards in DMF-Einheiten angegeben.

Überraschenderweise erlaubt es das erfindungsgemäße Verfahren auch, das Probe-/Reagenzvolumenverhältnis von 0,1, das nach dem Verfahren von Johnson et al. üblich ist, stark herabzusetzen, ohne daß bei gleichem Meßintervall und Inkubation bei gleicher Temperatur das Meßsignal mit einer definierten Menge an einem als Probe eingesetzten Fructosaminanalogon signifikant im Vergleich zu der von Johnson et al. beschriebenen Methode kleiner wird. Wird als Fructosaminanalogon z. B. DMF verwendet, so kann in diesem Fall das Probe-/Reagenzvolumenverhältnis auf 0,02 herabgesetzt werden, ohne die Empfindlichkeit der Messung zu verringern.

Das erfindungsgemäße Verfahren zur Bestimmung von Fructosamin in Blut oder von Blut abgeleiteten Proben, unter Vermeidung des Proteinmatrixeffekte mit Hilfe eines oder mehrerer enzymatischer oder/und nichtenzymatischer Oxidationsmittel, Lipase sowie eines oder mehrerer Detergentien, läßt sich nicht nur in Lösung durchführen. Es ist auch ohne weiteres auf Bestimmungsverfahren mittels trockenchemischer Testträger übertragbar. Das oder die enzymatischen oder/und nichtenzymatischen Oxidationsmittel, Lipase sowie ein oder mehrere Detergentien, gegebenenfalls Salze eventuell mit weiteren Hilfsstoffen werden hierzu in an sich bekannter Weise auf feste Träger aufgebracht. Geeignete feste Träger sowie Verfahren zum Aufbringen dieser Stoffe bzw. Stoffgemische auf solche Träger sind dem Fachmann bekannt. Als Trägermaterialien eignen sich beispielsweise alle möglichen saugfähigen Materialien, wie beispielsweise Papiere, Vliese usw.. Die aufzubringenden Stoffe können in eine oder mehrere Imprägnierlösungen aufgenommen werden. Mit diesen Lösungen werden die Träger imprägniert oder besprüht. Anschließend wird getrocknet.

Eine andere Möglichkeit wäre, das oder die Oxidationsmittel,Lipase sowie Detergentien und gegebenenfalls Salze sowie eventuell weitere Hilfsstoffe in Reagenzfilme einzubringen. Hierzu werden die Substanzen bzw. Substanzgemische z. B. entsprechend Verfahren gemäß DE-PS 1 598 153 oder DE-OS 2 910 134 zu Reagenzfilmen verarbeitet.

Zur Beseitigung störender unspezifisch reduzierend wirkender und trübungsverursachender Probenbestandteile wird die zu messende Probe zunächst mit einem Träger in Verbindung gebracht, der das oder die Oxidationsmittel, Lipase sowie ein oder mehrere Detergentien und gegebenenfalls Salze enthält. Nach ausreichendem Kontakt wird die vorbehandelte Probe in eine Schicht überführt, welche die weiteren zur Farbreaktion erforderlichen Reaktionsbestandteile enthält. Die dabei bewirkte Farbänderung wird in bekann-

EP 0 250 991 B1

ter Weise photometrisch, z. B. reflektometrisch gemessen. In Bezug auf die Zeitintervalle für die Vorreaktion und für die Farbreaktion gelten die oben gemachten Ausführungen.

Ein weiterer Gegenstand der Erfindung ist ein Mittel zur spezifischen Bestimmung des Serumfructosamingehaltes in Blut oder von Blut abgeleiteten Proben unter Vermeidung des Proteinmatrixeffektes, welches dadurch gekennzeichnet ist, daß es aus einem Reagenz enthaltend enzymatische oder/und nichtenzymatische Oxidationsmittel, Lipase und Detergenzien, einem Umpufferungsreagenz mit einem Puffer, der einen pH-Wert im Bereich von 10,5 - 12,5 aufweist und einem Redoxfarbreagenz zum Nachweis des Fructosamins besteht. Dieses Reagenzgemisch enthält alle zur Durchführung des erfindungsgemäßen Verfahrens notwendigen Bestandteile.

Gegebenenfalls enthält das Mittel zusätzlich Salze starker Säuren sowie gegebenenfalls übliche Zusatzstoffen in einem Puffer mit annähernd neutralem pH-Wert.

Unter enzymatischen Oxidationsmitteln sind insbesondere Ascorbat-Oxidase, Bilirubin-Oxidase und Uricase und unter nichtenzymatischen Oxidationsmitteln sind besonders Hypochlorit und hypochloritliefernde Verbindungen zu verstehen.

Die in dem erfindungsgemäßen Mittel enthaltenen Detergentien können anionisch oder nichtionisch sein. Während als anionische Detergentien vor allem Alkali- oder Erdalkalisalze von Gallensäuren und ihrer Konjugate bevorzugt werden, steht für nichtionische Detergentien eine breite Palette von Detergentien zur Auswahl. Als geeignet haben sich vor allem lineare oder verzweigtkettige Alkyl- oder Alkylaryl-Alkohol-Polyglycoläther mit 8 - 20 C-Atomen im Alkoholteil und 4 bis 15 Glycoleinheiten pro Molekül erwiesen. Die zugesetzten Detergentien können jedoch zusätzlich auch kationische Detergentien sein, wie beispielsweise Oxyethylalkylammoniumphosphat (z. B. Dehyquart[R]SP der Fa. Henkel).

Ganz besonders wirksam ist ein Gemisch von enzymatischen oder/und nichtenzymatischen Oxidationsmitteln, wenn es Chloramin T und gleichzeitig ein kationisches Detergens, beispielsweise Dehyquart[R]SP enthält.

Als Salze starker Säuren haben sich für das erfindungsgemäße Reagenz Alkali- oder Erdalkalisalze von Salz- oder Schwefelsäure als geeignet gezeigt. Besonders bevorzugt werden Kalium- oder Natriumchlorid.

Der zur Erzielung eines annähernd neutralen pH-Wertes des erfindungsgemäßen Reagenzes erforderliche Puffer weist einen pH-Wert in dem Bereich von pH 6 - 9, vorzugsweise pH 7 - 8,5, ganz besonders bevorzugt pH 7,5 - 8,0 auf. Die Pufferkonzentration beträgt vorzugsweise 10 - 100 mmol/l, ganz besonders bevorzugt 20 - 70 mmol/l. Als besonders vorteilhaft hat sich wässriger Kaliumphosphatpuffer erwiesen.

Erfindungsgemäßes Verfahren und Reagenzgemisch sind in den folgenden Beispielen weiter erläutert.

Beispiel 1

Fructosaminbestimmung

A) Reagenzienzusammensetzung

6

a) Reagenz I (für den ersten Inkubationsschritt)

| Komponente | Konzentration |
|---|---|
| Kaliumphosphatpuffer (pH 8.0) | 50 mmol/l |
| Kaliumchlorid | 50 mmol/l |
| Natrium-Cholat | 5 mmol/l |
| Oxetal$^R$ ID 104 | 0,25 % |
| Produkt RT 240$^R$ | 3 % |
| Uricase | 4 U/ml |
| Bilirubin-Oxidase | 0,1 U/ml |
| Lipase | 2 U/ml |
| Ascorbat-Oxidase | 10 U/ml |
| Peroxidase | 2 U/ml |
| Katalase | 1000 U/ml |

b) Reagenz II (für den zweiten Inkubationsschritt)

| Komponente | Konzentration |
|---|---|
| Natriumcarbonatpuffer (pH 10.9) | 200 mmol/l |
| Nitroblautetrazoliumsalz (NBT) | 0,5 mmol/l |

B) Testdurchführung

Wellenlänge: 546 nm
Temperatur: 37° C
Schichtdicke: 10 mm (Halbmikroküvette)

In Küvetten pipettieren:

| | Probe (P) | Reagenzleerwert (RL) |
|---|---|---|
| Reagenz I | 0,500 ml | 0,500 ml |
| Probe | 0,020 ml | - |
| dest. Wasser | - | 0,020 ml |

5 Minuten inkubieren, anschließend zumischen:

7

| Reagenz II | 0,500 ml | 0,500 ml |
|---|---|---|

erneut inkubieren und Kinetik der Farbstoffbildung ($\Delta E_p$ bzw. $\Delta E_{RL}$), innerhalb eines bestimmten Zeitintervalls $\Delta t$ (1 bis 15 min.) nach Zugabe von Reagenz II messen.

$$\Delta E = (\Delta E_p - \Delta E_{RL})/\Delta t$$

In entsprechender Weise wie vorstehend für die Probe ausgeführt, wird eine Standardlösung (S) mit bekanntem DMF-Gehalt vermessen. Aus den Meßwerten wird $\Delta E_{Standard}$ in folgender Weise ermittelt:

$$\Delta E_{Standard} = (\Delta E_S - \Delta E_{RL})/\Delta t$$

$$\text{Konzentration in der Probe} = \frac{\Delta E}{\Delta E_{Standard}}$$

Die Konzentration in der Probe wird in "1-Desoxymorpholinofructose-Einheiten" (DMF-Einheiten) angegeben. Zur Kalibrierung wurde der Eichstandard aus dem Fructosamin-Test der Fa. Roche, Basel, Schweiz (Art.-Nr. 07-1121-7) verwendet.

Eine Darstellung des Extinktionsverlaufs beim Testansatz nach dem erfindungsgemäßen Verfahren (Kurve 1) im Vergleich zur Methode von Johnson et al., Clin. Chim. Acta (1982) 127, 87 - 95 (Kurve 2), wobei der Eichstandard aus dem Fructosamintest der Fa. Roche, Basel, Schweiz als Probenmaterial diente, ist in Abb. 1 wiedergegeben.

Beispiel 2

Linearität

Zur Überprüfung der Linearität der Extinktionsänderung nach dem erfindungsgemäßen Verfahren wurde ein Humanserum mit 1-Deoxy-1-morpholinofructose (DMF) stufenweise aufgestockt und der Test entsprechend Beispiel 1 durchgeführt.

Wie aus Abb. 2 ersichtlich, ist die Extinktionsänderung/Minute bis mindestens 10 mmol/l linear proportional der DMF-Konzentration in der Probe.

Beispiel 3

Bilirubin-Störung

Zur Untersuchung des Einflusses von Bilirubin auf das Meßsignal im erfindungsgemäßen Verfahren zur Bestimmung von Fructosamin wurde ein Humanserum mit Bilirubin bis zu einer Konzentration von 12 mg/dl stufenweise aufgestockt und der Test wie in Beispiel 1 beschrieben durchgeführt.

Wie aus Abb. 3 zu ersehen, führt bei der Fructosamin-Bestimmung nach dem Verfahren von Johnson et al., Clin. Chim. Acta (1982) 127, 87 - 95 (Kurve 1), bereits eine Bilirubin-Konzentration von 2 mg/dl zu einem um ca. 40 % erhöhten Meßsignal, während es im erfindungsgemäßen Testverfahren (Kurve 2) bis zu mindestens 12 mg/dl Bilirubin völlig unbeeinflußt bleibt.

Beispiel 4

Harnsäure-Störung

Zur Bestimmung des Einflusses von Harnsäure auf das Meßsignal wurde Humanserum mit verschiedenen Mengen an Harnsäure aufgestockt und einmal nach dem Verfahren von Johnson et al., Clin. Chim. Acta (1982) 127, 87 - 95, zum anderen nach der erfindungsgemäßen Methode analog Beispiel 1 ein E/t-Diagramm aufgenommen.

| $\Delta$E/5 min (% Wiederfindung) | | | |
|---|---|---|---|
| Probe | Harnsäure (mg/dl) | Methode nach Johnson et al. | Erfindungsgemäße Methode |
| 1 | 6,9 | 0,082 (100 %) | 0,081 (100 %) |
| 2 | 13,7 | 0,093 (113 %) | 0,082 (101 %) |
| 3 | 17,8 | 0,097 (118 %) | 0,080 ( 99 %) |
| 4 | 24,2 | 0,104 (127 %) | 0,081 (100 %) |
| 5 | 30,0 | 0,107 (130 %) | 0,083 (102 %) |

Während beim Test nach Johnson et al. bereits bei einer Harnsäure-Konzentration von 13,7 mg/dl ein gegenüber dem Ausgangswert (6,9 mg Harnsäure/dl) um 13 % erhöhtes Signal gefunden wurde, bleibt der Test gemäß dem erfindungsgemäßen Verfahren von Harnsäure bis zu Konzentrationen von ca. 30 mg/dl praktisch unbeeinflußt.

Beispiel 5

Ascorbinsäure-Störung

Zur Bestimmung des Einflusses von Ascorbinsäure auf das Meßsignal wurde Humanserum mit verschiedenen Mengen an Ascorbinsäure aufgestockt und einmal nach dem Verfahren von Johnson et al., Clin. Chim. Acta (1982) 127, 87 - 95, zum anderen nach der erfindungsgemäßen Methode analog Beispiel 1 ein E/t-Diagramm aufgenommen.

| $\Delta$mE/min (% Wiederfindung) | | | |
|---|---|---|---|
| Probe | Ascorbinsäure (mg/l) | Methode nach Johnson et al. | Erfindungsgemäße Methode |
| 1 | 0 | 16,2 (100 %) | 16,5 (100 %) |
| 2 | 10 | 15,6 ( 96,3) | 16,5 (100 %) |
| 3 | 20 | 15,4 ( 95,1) | 17,0 (103 %) |
| 4 | 30 | 14,8 ( 91,7) | 17,5 (106 %) |
| 5 | 50 | 14,2 ( 87,7) | 17,0 (103 %) |

Während beim Test nach Johnson et al. mit steigender Ascorbinsäurekonzentration die Wiederfindungsrate zunehmend sinkt, bleibt der Test gemäß dem erfindungsgemäßen Verfahren bis zu Konzentrationen von ca. 50 mg/l praktisch unbeeinflußt.

Beispiel 6

Aufklarung lipämischer Seren

Im Testansatz entsprechend Beispiel 1 wurde als Probe stark hyperlipämisches Serum (Triglyceride, 2000 mg/dl) eingesetzt.

Wie aus Abb. 4 ersichtlich, wird im ersten Inkubationsschritt bereits nach 5 Minuten eine vollständige und dauerhafte Trübungsbeseitigung erreicht.

Beispiel 7

Medikamentenstörung

Zur Bestimmung des Einflusses von Medikamenten auf das Meßsignal wurde Humanserum mit verschiedenen Mengen unterschiedlicher Medikamente oder Medikamentenabbauprodukten aufgestockt und nach dem Verfahren von Johnson et al., Clin. Chim. Acta (1982) 127, 87 - 95 sowie nach dem erfindungsgemäßen Verfahren analog Beispiel 1 ein E/t-Diagramm aufgenommen.

| ΔmE/min (% Wiederfindung) | | | |
|---|---|---|---|
| Medikament bzw. Abbauprodukt | Konzentration im Serum | Test nach Johnson et al. | Erfindungs- gemäßer Test |
| | 0 | 17,4 (100 %) | 15,6 (100 %) |
| | 1 mg/l | 18,4 (106 %) | 16,1 (103 %) |
| α -Methyldopa | 10 mg/l | 22,6 (130 %) | 17,2 (110 %) |
| | 100 mg/l | nicht mehr meßbar | 22,5 (144 %) |
| | 0 | 17,4 (100 %) | 15,6 (100 %) |
| Gentisinsäure | 1 mg/l | 19,2 (110 %) | 15,9 (102 %) |
| Na-Salz | 10 mg/l | 24,8 (143 %) | 16,7 (107 %) |
| | 100 mg/l | ca. 390 (2000%) | 22,2 (142 %) |

Beispiel 8

Einfluß der Proben-Gesamtproteinmenge auf das Meßsignal

Es wurden in einem Modellversuch Lösungen mit verschiedenen Konzentrationen an Rinderserumalbumin (RSA) in physiologischer Kochsalzlösung hergestellt, einmal ohne Zusatz von 1-Deoxymorpholinofructose (DMF) für die Leerwertermittlung, zum anderen unter Zusatz einer konstanten Menge an DMF (2,5 mmol/l). Der Test wurde sowohl nach der Methode von Johnson et al., Clin. Chim. Acta (1982) 127, 87 - 95, als auch nach dem erfindungsgemäßen Verfahren entsprechend Beispiel 1 durchgeführt.

EP 0 250 991 B1

| Δ mE/min (% Wiederfindung) | | |
|---|---|---|
| RSA ohne DMF (g/l) | Test nach Johnson et al. | Test nach dem erfindungs- gemäßen Verfahren |
| 0 | 0,0 | 2,8 |
| 20 | 12,1 | 4,9 |
| 40 | 17,8 | 7,0 |
| 60 | 18,7 | 8,3 |
| 80 | 21,9 | 9,4 |
| 100 | 23,8 | 10,8 |

| Differenz: | | |
|---|---|---|
| RSA mit DMF | $(\Delta mE/min)_{mit\ DMF}$ - $(\Delta mE/min)_{ohne\ DMF}$ (% Wiederfindung) | |
| 0 | 15,9 (100 %) | 12,3 (100 %) |
| 20 | 11,1 ( 70 %) | 12,1 ( 98 %) |
| 40 | 9,8 ( 62 %) | 12,2 ( 99 %) |
| 60 | 8,8 ( 55 %) | 11,2 ( 91 %) |
| 80 | 6,2 ( 39 %) | 11,1 ( 90 %) |
| 100 | 4,3 ( 27 %) | 12,0 ( 97 %) |

Wie aus der Tabelle zu ersehen, wird das von DMF in der Probe erzeugte Meßsignal im Test nach Johnson et al. bei steigenden Proteinkonzentrationen sehr stark vermindert, während es im Test nach dem erfindungsgemäßen Verfahren bis zu mindestens 100 g RSA/l praktisch unbeeinflußt von der Proteinmenge ist.

Beispiel 9

Fructosaminbestimmung

A) Reagenzienzusammensetzung

a) Reagenz I (für den ersten Inkubationsschritt)

| Komponente | Konzentration |
|---|---|
| Kaliumphosphatpuffer (pH 8.0) | 50 mmol/l |
| Kaliumchlorid | 50 mmol/l |
| Natrium-Cholat | 7 mmol/l |
| Lutensol$^R$ ON60 (BASF) | 2,4 % |
| Uricase | 4 U/ml |
| Bilirubin-Oxidase | 0,1 U/ml |
| Lipase | 2 U/ml |
| Ascorbat-Oxidase | 10 U/ml |
| Peroxidase | 2 U/ml |

b) Reagenz II (für den zweiten Inkubationsschritt)

| Komponente | Konzentration |
|---|---|
| Natriumcarbonatpuffer (pH 10.9) | 200 mmol/l |
| Nitroblautetrazoliumsalz (NBT) | 0,5 mmol/l |

B) Testdurchführung

Die Tests wurden an einem Hitachi 704 Analysenautomaten durchgeführt.

Wellenlänge: 546 nm
Temperatur: 37° C
Schichtdicke: 10 mm (Halbmikroküvette)

In Küvetten pipettieren:

| | Probe (P) | Reagenzleerwert (RL) |
|---|---|---|
| Reagenz I | 0,175 ml | 0,175 ml |
| Probe | 0,007 ml | - |
| dest. Wasser | - | 0,007 ml |

5 Minuten inkubieren, anschließend zumischen:

| Reagenz II | 0,175 ml | 0,175 ml |
|---|---|---|

erneut inkubieren und Kinetik der Farbstoffbildung ($\Delta E_p$ bzw. $\Delta E_{RL}$), innerhalb eines bestimmten Zeitintervalls $\Delta t$ (8 bis 10 min.) nach Zugabe von Reagenz II messen.

Die Konzentrationen an Fructosamin wurden analog zu Beispiel 1 als DMF-Einheiten ermittelt und in mmol/l auf der Ordinate der Abb. 5a aufgetragen. Diese Ergebnisse wurden gegen die Meßwerte aufgetragen, die durch Messen von Furosin mit der HPLC-Bezugsmethode (J. Clin. Chem. Clin. Biochem. 19 - (1981), S. 81 - 87) erhalten wurden. Als HPLC-Meßwerte wurden jeweils relative Peakflächeneinheiten verwendet.

In Abb. 5b sind die Ergebnisse für eine analoge Fructosaminbestimmung dargestellt, wobei zusätzlich 1,2 % Dehyquart[R]SP (Fa. Henkel) in Reagenz I enthalten ist.

Abb. 5c gibt die Meßergebnisse wieder für eine analoge Fructosaminbestimmung, bei der zusätzlich 250 $\mu$M Chloramin T in Reagenz I enthalten ist.

Abb. 5d schließlich gibt die Meßergebnisse wieder für eine analoge Fructosaminbestimmung, wobei sowohl 1,2 % Dehyquart[R]SP als auch 250 $\mu$M Chloramin T in Reagenz I zusätzlich enthalten sind.

Den Diagrammen 5a - d ist zu entnehmen, daß insbesondere die Kombination von Chloramin T und Dehyquart[R]SP den Achsenabschnitt deutlich reduziert.

**Patentansprüche**

1. Verfahren zur spezifischen Bestimmung des Serumfructosamingehaltes in Blut oder von Blut abgeleiteten Proben durch Umsetzung mit einem Redoxfarbreagenz und Messung der dabei bewirkten Farbänderung unter Vermeidung des Proteinmatrixeffektes, dadurch gekennzeichnet, daß vor der Farbreaktion die Probe mit einem oder mehreren enzymatischen oder/und nichtenzymatischen Oxidationsmitteln, Lipase und einem oder mehreren Detergenzien bei einem pH zwischen 6 und 9 behandelt, anschließend ein pH-Wert zwischen 10 und 12 eingestellt und das Farbreagenz zugegeben wird.

2. Verfahren gemäß Anspruch 1, dadurch gekennzeichnet, daß als enzymatische Oxidationsmittel Ascorbat-Oxidase, Bilirubin-Oxidase oder/und Uricase eingesetzt wird.

3. Verfahren gemäß Anspruch 1, dadurch gekennzeichnet, daß als nichtenzymatisches Oxidationsmittel Hypochlorit oder eine hypochloritliefernde Verbindung eingesetzt werden.

4. Verfahren gemäß einem der Ansprüche 1 - 3, daduch gekennzeichnet, daß als nichtenzymatisches Oxidationsmittel N-Chlor-p-toluolsulfamid und als Detergenz Oxyethylammoniumphosphat zugesetzt wird.

5. Verfahren gemäß einem der Ansprüche 1 - 4, dadurch gekennzeichnet, daß zusätzlich Peroxidase oder/und Katalase zugesetzt wird.

6. Verfahren gemäß einem der Ansprüche 1 -5, dadurch gekennzeichnet, daß zusätzlich Alkali- oder Erdalkalisalze von Salz oder Schwefelsäure zugesetzt werden.

7. Verfahren gemäß Anspruch 1, dadurch gekennzeichnet, daß zur Einstellung des pH-Wertes zwischen 10 und 12 ein Puffer zugegeben wird, der einen pH-Wert im Bereich zwischen 10,5 und 12,5 aufweist.

8. Verfahren gemäß Anspruch 7, dadurch gekennzeichnet, daß gleichzeitig mit dem Puffer, der einen pH-Wert im Bereich von 10,5 bis 12,5 aufweist, das Farbreagenz zugegeben wird.

9. Mittel zur spezifischen Bestimmung des Serumfructosamingehaltes in Blut oder von Blut abgeleiteten Proben unter Vermeidung des Matrixeffektes, dadurch gekennzeichnet, daß es aus einem Reagenz enthaltend enzymatische oder/und nichtenzymatische Oxidationsmittel, Lipase und Detergenzien, einem Umpufferungsreagenz mit einem Puffer, der einen pH-Wert im Bereich von 10,5 bis 12,5 aufweist, und einem Redoxfarbreagenz zum Nachweis des Fructosamins besteht.

**10.** Mittel gemäß Anspruch 9, dadurch gekennzeichnet, daß es als enzymatische Oxidationsmittel Ascorbatoxidase, Bilirubin-Oxidase oder/und Uricase enthält.

**11.** Mittel gemäß Anspruch 9, dadurch gekennzeichnet, daß es als nichtenzymatisches Oxidationsmittel Hypochlorit oder eine hypochloritliefernde Verbindung enthält.

**12.** Mittel gemäß einem der Ansprüche 9 - 11 dadurch gekennzeichnet, daß es als nichtenzymatisches Oxidationsmittel N-chlor-p-toluolsulfamid und als kationisches Detergenz Oxyethylammoniumphosphat enthält.

**13.** Mittel gemäß einer der Ansprüche 9 - 12, dadurch gekennzeichnet, daß es zusätzlich Peroxidase oder/und Katalase enthält.

**14.** Mittel gemäß einem der Ansprüche 9 - 13, dadurch gekennzeichnet, daß es zusätzlich Alkali- oder Erdalkalisalze von Salz- oder Schwefelsäure enthält.

**Claims**

**1.** Process for the specific determination of the serum fructosamine content in blood or samples derived from blood by reaction with a redox colour reagent and measurement of the colour change thereby brought about with avoidance of the protein matrix effect, characterised in that, before the colour reaction, the sample is treated with one or more enzymatic or/and non-enzymatic oxidation agents, lipase and one or more detergents at a pH between 6 and 9, subsequently adjusts a pH value between 10 and 12 and adds the colour reagent thereto.

**2.** Process according to claim 1, characterised in that, as enzymatic oxidation agent, there is used ascorbate oxidase, bilirubin oxidase or/and uricase.

**3.** Process according to claim 1, characterised in that, as non-enzymatic oxidation agents, there are used hypochlorite or a hypochlorite-providing compound.

**4.** Process according to one of claims 1 - 3, characterised in that, as non-enzymatic oxidation agent, there is added N-chloro-p-toluene sulphamide and, as detergent, oxyethyl ammonium phosphate.

**5.** Process according to one of claims 1 - 4, characterised in that peroxidase or/and catalase is additionally added.

**6.** Process according to one of claims 1 - 5, characterised in that alkali metal or alkaline earth metal salts of hydrochloric or sulphuric acid are additionally added.

**7.** Process according to claim 1, characterised in that, for the adjustment of the pH value between 10 and 12, a buffer is added which has a pH value in the range between 10.5 and 12.5.

**8.** Process according to claim 7, characterised in that the colour reagent is added simultaneously with the buffer which has a pH value in the range of 10.5 to 12.5.

**9.** Agent for the specific determination of the serum fructosamine content in blood or samples derived from blood with the avoidance of the matrix effect, characterised in that it consists of a reagent containing enzymatic or/and non-enzymatic oxidation agents, lipase and detergents, a rebuffering reagent with a buffer which has a pH value in the range of 10.5 to 12.5 and a redox colour reagent for the detection of fructosamine.

**10.** Agent according to claim 9, characterised in that, as enzymatic oxidation agent, it contains ascorbate oxidase, bilirubin oxidase or/and uricase.

**11.** Agent according to claim 9, characterised in that, as non-enzymatic oxidation agent, it contains hypochlorite or a hypochlorite-providing compound.

14

**12.** Agent according to one of claims 9 - 11, characterised in that, as non-enzymatic oxidation agent, it contains N-chloro-p-toluene sulphamide and, as cationic detergent, oxyethyl ammonium phosphate.

**13.** Agent according to one of claims 9 - 12, characterised in that it additionally contains peroxidase or/and catalase.

**14.** Agent according to one of claims 9 - 13, characterised in that it additionally contains alkali metal or alkaline earth metal salts of hydrochloric or sulphuric acid.

**Revendications**

**1.** Procédé pour la détermination spécifique de la teneur en fructosamine sérique dans le sang ou dans des échantillons dérivés du sang, par réaction avec un réactif colorant redox et mesure du changement de couleur ainsi obtenu, en évitant les effets de la matrice protéique, caractérisé en ce qu'avant la réaction colorimétrique, on traite l'échantillon avec un ou plusieurs agents oxydants enzymatiques ou non enzymatiques, des lipases et un ou plusieurs détergents, à un pH compris entre 6 et 9, puis on ajuste le pH à une valeur comprise entre 10 et 12, et l'on introduit le réactif colorant.

**2.** Procédé selon la revendication 1, caractérisé en ce que, comme agent oxydant enzymatique, on utilise l'ascorbate-oxydase, la bilirubine-oxydase et/ou l'uricase.

**3.** Procédé selon la revendication 1, caractérisé en ce que, comme agent oxydant non enzymatique, on utilise l'hypochlorite ou un composé libérant de l'hypochlorite.

**4.** Procédé selon l'une quelconque des revendications 1 à 3, caractérisé en ce que, comme agent oxydant non enzymatique, on utilise le N-chloro-p-toluènesulfamide et comme détergent, le phosphate d'oxyéthylammonium.

**5.** Procédé selon l'une quelconque des revendications 1 à 4, caractérisé en ce que l'on ajoute en plus de la peroxydase et/ou de la catalase.

**6.** Procédé selon l'une quelconque des revendications 1 à 5, caractérisé en ce que l'on ajoute en plus des sels alcalins ou alcalino-terreux de l'acide chlorhydrique ou de l'acide sulfurique.

**7.** Procédé selon la revendication 1, caractérisé en ce que, pour ajuster la valeur du pH entre 10 et 12, l'on ajoute un tampon qui présente un pH dans la gamme de 10,5 à 12,5.

**8.** Procédé selon la revendication 7, caractérisé en ce que, en même temps que le tampon, qui présente un pH dans la gamme de 10,5 à 12,5, on ajoute le réactif colorant.

**9.** Agent pour la détermination spécifique de la teneur en fructosamine sérique dans le sang ou dans des échantillons dérivés du sang, en évitant les effets de la matrice, caractérisé en ce qu'il est constitué par un réactif contenant des agents oxydants enzymatiques et/ou non enzymatiques, des lipases et des détergents, un réactif tampon contenant un tampon qui présente un pH compris entre 10,5 et 12,5, et un réactif colorant redox, pour la détermination de la fructosamine.

**10.** Agent selon la revendication 9, caractérisé en ce qu'il contient, comme agent oxydant enzymatique, l'ascorbate-oxydase, la bilirubine-oxydase et/ou l'uricase.

**11.** Agent selon la revendication 9, caractérisé en ce qu'il contient, comme agent oxydant non enzymatique, l'hypochlorite ou un composé libérant de l'hypochlorite.

**12.** Agent selon l'une quelconque des revendications 9-11, caractérisé en ce que, comme agent oxydant non enzymatique, il contient le N-chloro-p-toluènesulfamide et comme détergent cationique, le phosphate d'oxyéthylammonium.

**13.** Agent selon l'une quelconque des revendications 9-12, caractérisé en ce qu'il contient en outre de la peroxydase et/ou de la catalase.

**14.** Agent selon l'une quelconque des revendications 9-13, caractérisé en ce qu'il contient en outre des sels alcalins ou alcalino-terreux de l'acide chlorhydrique ou de l'acide sulfurique.